# EUROPEAN PATENT APPLICATION

(11) **EP 4 613 229 A1**
(43) Date of publication of application: **10.09.2025**
(21) Application number: 25157551.0
(22) Date of filing: 13.02.2025
(51) Int. Cl.: A61B 34/37, A61B 34/30, A61B 34/00

(54) **ROBOTIC SURGICAL SYSTEM, CONTROL METHOD FOR ROBOTIC SURGICAL SYSTEM, PROGRAM, AND STORAGE MEDIUM**

(30) Priority: 01.03.2024 JP 2024030882
(71) Applicant: KAWASAKI JUKOGYO KABUSHIKI KAISHA, Kobe-shi, Hyogo 650-8670 (JP)
(72) Inventor: KOBAYASHI, Ayataka, Kobe-shi, Hyogo, 650-8670 (JP); KODAMA, Kazuki, Kobe-shi, Hyogo, 650-8670 (JP)
(74) Representative: Hoffmann Eitle

(57) **Abstract**

A robotic surgical system (500) includes a controller (310) configured or programmed to perform a process to move a control point set at a virtual position (P0) offset ahead of a distal end of an endoscope (3) in an axial direction of the endoscope (3) by a distance corresponding to a given distance (ΔX) when an operation unit (110) receives an operation to move the endoscope (3) by the given distance (ΔX).

## Description

### BACKGROUND OF THE INVENTION

### Field of the Invention

The present disclosure relates to a robotic surgical system, a control method for a robotic surgical system, a program, and a storage medium.

### Description of the Background Art

Conventionally, a robotic surgical system including a robot arm that supports a surgical instrument is known. U.S. Patent No. 8,004,229 discloses a robotic surgical system including a manipulator that supports a surgical instrument or an endoscope, a master controller that operates the manipulator, and a controller that controls the operation of the manipulator. In U.S. Patent No. 8,004,229, the controller receives an operation input from the master controller and controls the surgical instrument or the endoscope supported by the manipulator to move around a pivot position based on the received operation. The pivot position is the incision site of a patient.

When the operation of the manipulator is controlled such that the endoscope rotates around the pivot position as a fulcrum as in U.S. Patent No. 8,004,229, the field of view of the endoscope may change significantly depending on the pivot position even with a small movement of the manipulator. Furthermore, the pivot position is set at the incision site of the patient, and the endoscope rotates around the incision site as a fulcrum. In particular, when a distance between the distal end of the endoscope and the incision site is short, the rotation angle of the endoscope around the incision site as the fulcrum becomes large even when the distal end of the endoscope is moved slightly, and the field of view of the endoscope moves suddenly and largely. Therefore, it is desired to reduce or prevent large movement of the field of view of the endoscope.

### SUMMARY OF THE INVENTION

The present disclosure is intended to provide a robotic surgical system, a control method for a robotic surgical system, a program, and a storage medium each capable of reducing or preventing large movement of the field of view of an endoscope.

A robotic surgical system according to a first aspect of the present disclosure includes a surgical apparatus including a first robot arm to support an endoscope, an operation apparatus including an operation unit to receive an operation for the endoscope, and a controller configured or programmed to perform a process to move a control point set at a virtual position offset ahead of a distal end of the endoscope in an axial direction of the endoscope by a distance corresponding to a given distance when the operation unit receives an operation to move the endoscope by the given distance. The term "distal end of the endoscope" indicates a broader concept including the distal end itself of the endoscope and a portion in the vicinity of the distal end.

In the robotic surgical system according to the first aspect of the present disclosure, the controller is configured or programmed to perform the process to move the control point set at the virtual position offset ahead of the distal end of the endoscope in the axial direction of the endoscope by the distance corresponding to the given distance when the operation unit receives the operation to move the endoscope by the given distance. Accordingly, for example, when the distal end of the endoscope is moved in an arcuate shape with a reference point as a fulcrum, the movement amount of the distal end of the endoscope is smaller when the control point set at the virtual position offset ahead of the distal end of the endoscope is moved by the distance corresponding to the given distance than when the distal end of the endoscope is moved by the distance corresponding to the given distance. Consequently, it is possible to reduce or prevent large movement of the field of view of the endoscope.

A control method for a robotic surgical system according to a second aspect of the present disclosure includes receiving, by an operation unit configured to receive an operation for an endoscope supported by a robot arm, an operation to move the endoscope by a given distance, and moving a control point set at a virtual position offset ahead of a distal end of the endoscope in an axial direction of the endoscope by a distance corresponding to the given distance. The term "distal end of the endoscope" indicates a broader concept including the distal end itself of the endoscope and a portion in the vicinity of the distal end.

As described above, the control method for a robotic surgical system according to the second aspect of the present disclosure includes moving the control point set at the virtual position offset ahead of the distal end of the endoscope in the axial direction of the endoscope by the distance corresponding to the given distance. Accordingly, for example, when the distal end of the endoscope is moved in an arcuate shape with a reference point as a fulcrum, the movement amount of the distal end of the endoscope is smaller when the control point set at the virtual position offset ahead of the distal end of the endoscope is moved by the distance corresponding to the given distance than when the distal end of the endoscope is moved by the distance corresponding to the given distance. Consequently, it is possible to provide the control method for a robotic surgical system capable of reducing or preventing large movement of the field of view of the endoscope.

According to the present disclosure, it is possible to reduce or prevent large movement of the field of view of the endoscope.

The foregoing and other objects, features, aspects and advantages of the present disclosure will become more apparent from the following detailed description of the present disclosure when taken in conjunction with the accompanying drawings.

### BRIEF DESCRIPTION OF THE DRAWINGS

FIG. 1 is a diagram showing the configuration of a robotic surgical system according to an embodiment.
FIG. 2 is a diagram showing a display of a medical cart according to the embodiment.
FIG. 3 is a diagram showing the configuration of the medical cart according to the embodiment.
FIG. 4 is a diagram showing the configuration of a robot arm according to the embodiment.
FIG. 5 is a diagram showing an instrument.
FIG. 6 is a perspective view showing the configuration of an arm operation unit according to the embodiment.
FIG. 7 is a diagram for illustrating translational movement of the robot arm.
FIG. 8 is a diagram for illustrating rotational movement of the robot arm.
FIG. 9 is a diagram showing an endoscope.
FIG. 10 is a diagram showing a pivot position setting instrument.
FIG. 11 is a diagram showing operation units according to the embodiment.
FIG. 12 is a diagram showing a right-handed wrist according to the embodiment.
FIG. 13 is a diagram showing a left-handed wrist according to the embodiment.
FIG. 14 is a perspective view showing foot pedals according to the embodiment.
FIG. 15 is a control block diagram of the robotic surgical system according to the embodiment.
FIG. 16 is a control block diagram of the robot arm according to the embodiment.
FIG. 17 is a control block diagram of a positioner and the medial cart according to the embodiment.
FIG. 18 is a control block diagram of the operation unit according to the embodiment.
FIG. 19 is a diagram for illustrating a method for setting a pivot position.
FIG. 20 is a diagram for illustrating a method for calculating an axis value of each axis.
FIG. 21 is a diagram showing the pivot positions and tool center points of the instrument and the endoscope.
FIG. 22 is a diagram showing a state in which the distal ends of the instrument and the endoscope have been moved.
FIG. 23 is a diagram showing a state in which a virtual position offset ahead of the distal end of the endoscope has been moved.
FIG. 24 is a diagram showing a virtual plane set for the endoscope.
FIG. 25 is a flowchart for illustrating a control method for the robotic surgical system according to the embodiment.

### DESCRIPTION OF THE PREFERRED EMBODIMENTS

### Configuration of Robotic Surgical System

The configuration of a robotic surgical system 500 according to this embodiment is now described. The robotic surgical system 500 includes a surgical robot 100, a remote control apparatus 200, a vision unit 300, and an image processing unit 400. The remote control apparatus 200 is an example of an operation apparatus.

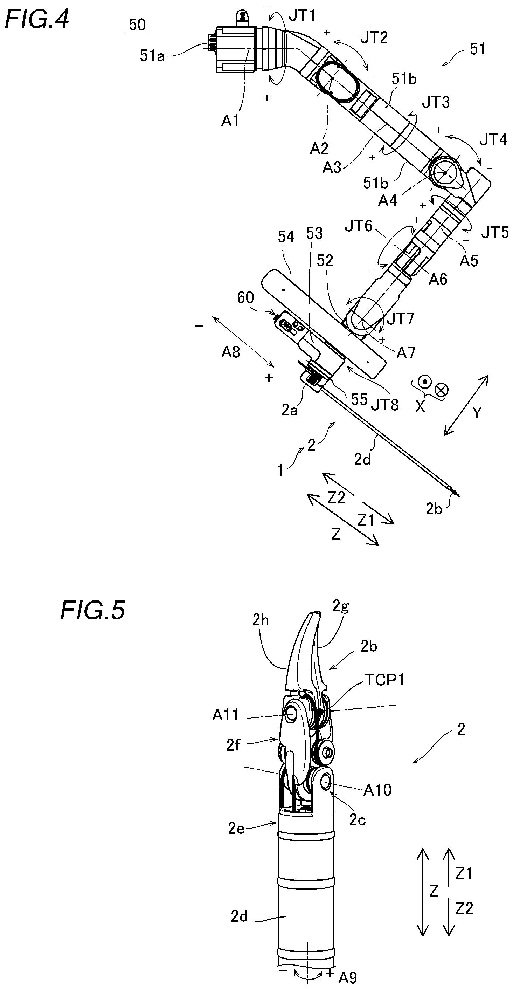

In this specification, as shown in FIG. 4, the longitudinal direction of a surgical instrument 1 is defined as a Z direction. The distal end side of the surgical instrument 1 is defined as a Z1 side, and the proximal end side of the surgical instrument 1 is defined as a Z2 side. A direction perpendicular to the Z direction is defined as an X direction. A direction perpendicular to the Z direction and the X direction is defined as a Y direction.

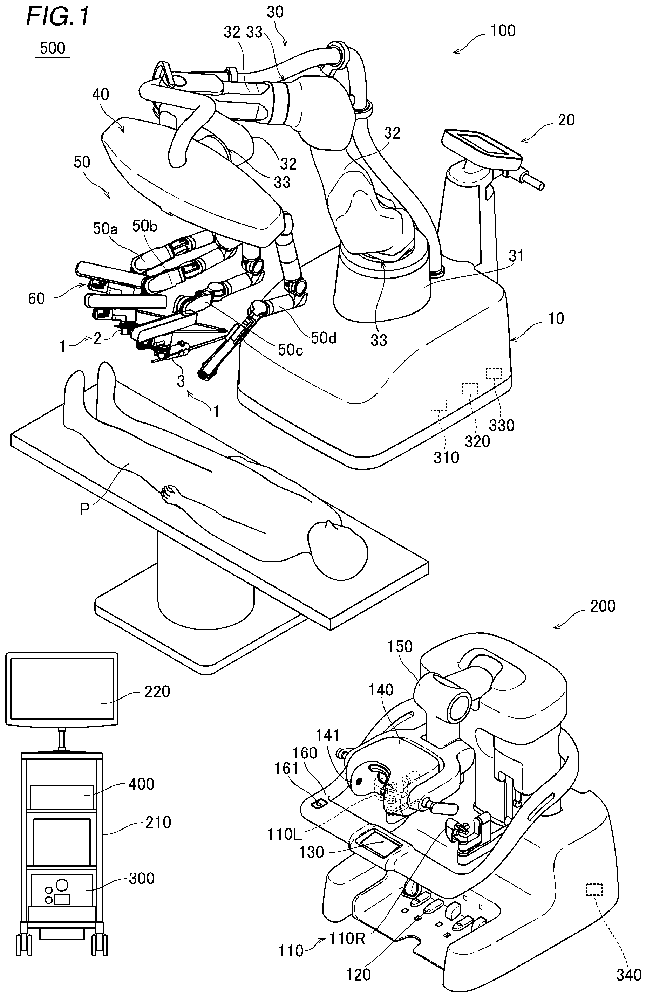

As shown in FIG. 1, the surgical robot 100 is arranged in an operating room. The remote control apparatus 200 is spaced apart from the surgical robot 100. The remote control apparatus 200 receives operations for the surgical instrument 1. Specifically, an operator such as a doctor inputs a command to the remote control apparatus 200 to cause the surgical robot 100 to perform a desired operation. The remote control apparatus 200 transmits the input command to the surgical robot 100. The surgical robot 100 operates based on the received command. The surgical robot 100 is arranged in the operating room that is a sterilized sterile field.

### Configuration of Surgical Robot

As shown in FIG. 1, the surgical robot 100 includes a medical cart 10, a cart positioner operation unit 20, a positioner 30, an arm base 40, a plurality of robot arms 50, and an arm operation unit 60 provided on each of the robot arms 50.

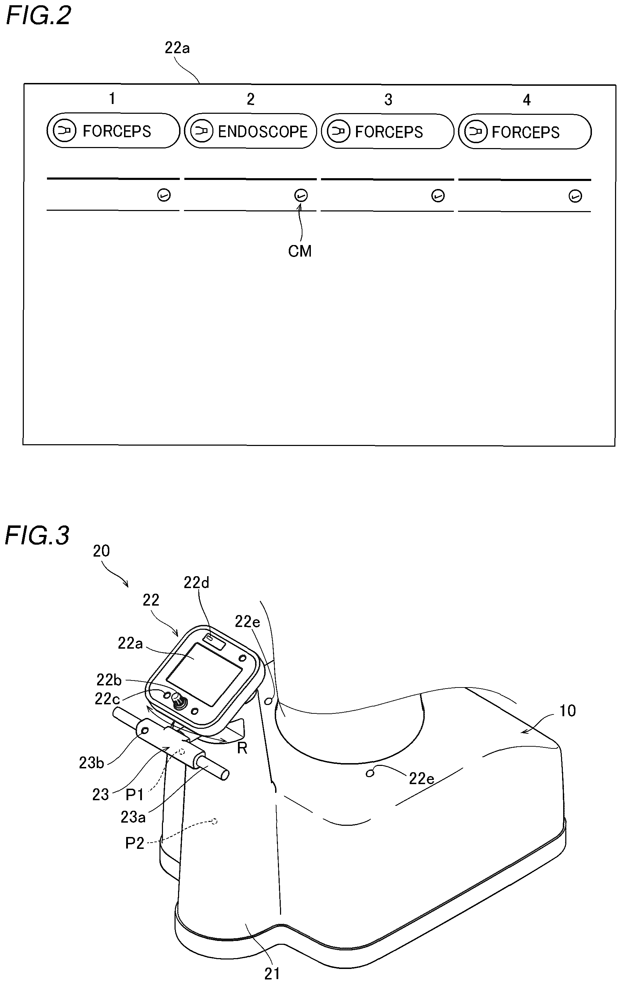

As shown in FIG. 3, the cart positioner operation unit 20 is supported by a cart positioner operation support 21 at the rear of the medical cart 10, and the medical cart 10 or the positioner 30 is moved by operating the cart positioner operation unit 20. The cart positioner operation unit 20 includes an input 22 and an operation handle 23. The input 22 receives operations to move the positioner 30, the arm base 40, and the plurality of robot arms 50 or change their postures mainly in order to prepare for surgery before the surgery. The medical cart 10 includes the operation handle 23.

As shown in FIG. 3, the input 22 includes a display 22a, a joystick 22b, an enable switch 22c, an error reset button 22d, and speakers 22e. The display 22a is a liquid crystal panel, for example. As shown in FIG. 2, the display 22a displays numbers corresponding to the plurality of robot arms 50. The display 22a also displays the type of surgical instrument 1 attached to each of the plurality of robot arms 50. A check mark CM indicating that a pivot position PP described below has been set is displayed on the display 22a.

As shown in FIG. 3, the joystick 22b is arranged in the vicinity of or adjacent to the display 22a of the input 22. The positioner 30 is moved three-dimensionally by selecting an operation mode displayed on the display 22a and operating the joystick 22b.

The enable switch 22c is arranged in the vicinity of or adjacent to the joystick 22b. The enable switch 22c enables or disables movement of the positioner 30. When the joystick 22b is operated while the enable switch 22c is pressed to enable movement of the positioner 30, the positioner 30 is moved.

The error reset button 22d resets errors in the robotic surgical system 500. The errors may be abnormal deviation errors, for example. The speakers 22e are arranged in pair. The pair of speakers 22e are arranged in the vicinity of or adjacent to the location of the positioner 30 in the medical cart 10.

The operation handle 23 is arranged in the vicinity of the display 22a. The operation handle 23 includes a throttle 23a that is gripped and twisted by an operator such as a nurse or a technician to operate movement of the medical cart 10. Specifically, the operation handle 23 is arranged below the input 22. As the throttle 23a is twisted from the near side to the far side, the medical cart 10 moves forward. As the throttle 23a is twisted from the far side to the near side, the medical cart 10 moves rearward. The speed of the medical cart 10 is changed according to a twisting amount of the throttle 23a. The operation handle 23 is rotatable to the left and right shown by an R direction, and the medical cart 10 is turned with rotation of the operation handle 23.

An enable switch 23b for enabling or disabling movement of the medical cart 10 is provided on the operation handle 23 of the cart positioner operation unit 20. When the throttle 23a of the operation handle 23 is operated while the enable switch 23b is pressed to enable movement of the medical cart 10, the medical cart 10 is moved.

As shown in FIG. 1, the positioner 30 includes a 7-axis articulated robot, for example. The positioner 30 is arranged on the medical cart 10. The positioner 30 adjusts the position of the arm base 40. The positioner 30 moves the position of the arm base 40 three-dimensionally.

The positioner 30 includes a base 31 and a plurality of links 32 coupled to the base 31. The plurality of links 32 are coupled to each other by joints 33.

The arm base 40 is attached to the distal end of the positioner 30. The proximal end of each of the plurality of robot arms 50 is attached to the arm base 40. Each of the plurality of robot arms 50 is able to take a folded and stored posture. The arm base 40 and the plurality of robot arms 50 are covered with sterile drapes and used. Moreover, each of the robot arms 50 supports the surgical instrument 1.

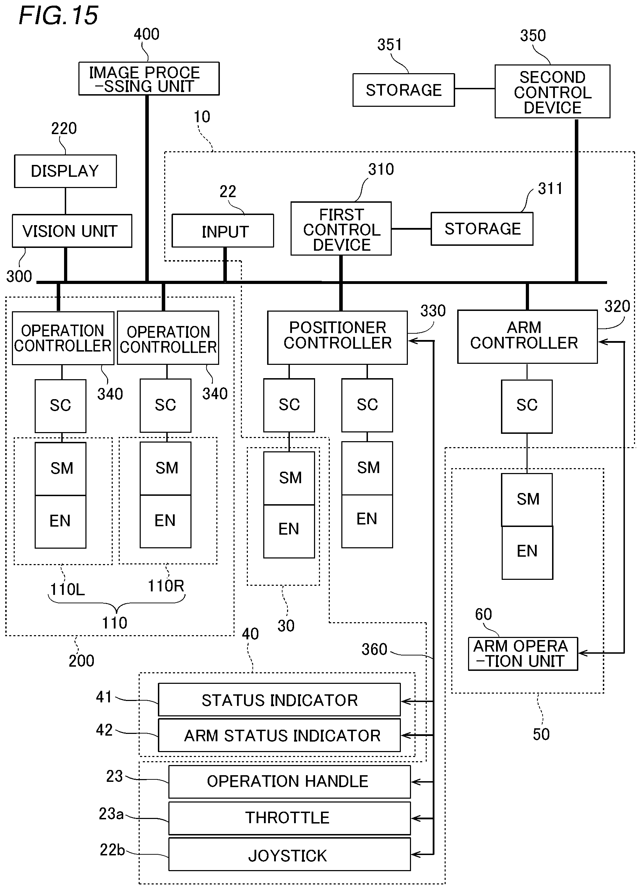

A status indicator 41 and an arm status indicator 42 that are shown in FIG. 15 are provided on the arm base 40. The status indicator 41 indicates the status of the robotic surgical system 500. The arm status indicator 42 indicates the statuses of the robot arms 50.

The plurality of robot arms 50 are arranged. Specifically, four robot arms 50a, 50b, 50c, and 50d are arranged. The robot arms 50a, 50b, 50c, and 50d have the same or similar configurations as each other.

As shown in FIG. 4, each robot arm 50 includes an arm portion 51, a first link 52, a second link 53, and a translation mechanism 54. The robot arm 50 includes joints JT1, JT2, JT3, JT4, JT5, JT6, JT7, and JT8. The joints JT1, JT2, JT3, JT4, JT5, JT6, and JT7 have A1, A2, A3, A4, A5, A6, and A7 axes as rotation axes, respectively. The joint JT8 has an A8 axis as a linear motion axis. The arm portion 51 includes a base 51a and links 51b.

The arm portion 51 includes a 7-axis articulated robot arm. The first link 52 is arranged at the distal end of the arm portion 51. The arm operation unit 60 described below is attached to the second link 53. The translation mechanism 54 is arranged between the first link 52 and the second link 53. A holder 55 that holds the surgical instrument 1 is arranged on the second link 53. The translation mechanism 54 translates the holder 55 to which the surgical instrument 1 is attached between a first position and a second position. The first position refers to an end position on the Z2 side in a range of movement of the holder 55 along the A8 axis by the translation mechanism 54. The second position refers to an end position on the Z1 side in the range of movement of the holder 55 along the A8 axis by the translation mechanism 54.

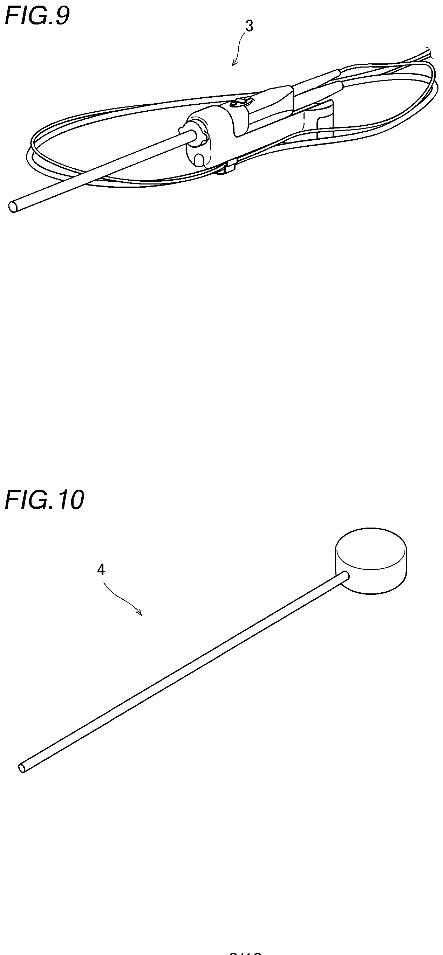

The surgical instrument 1 is attached to the distal end of each of the plurality of robot arms 50. The surgical instrument 1 includes a replaceable instrument 2, an endoscope 3 shown in FIG. 9 to capture an image of a surgical site, and a pivot position setting instrument 4 shown in FIG. 10 to set the pivot position PP, for example. The instrument 2 includes a driven unit 2a, an end effector 2b, and a wrist joint 2c and a shaft 2d shown in FIG. 5. The end effector 2b is connected to the distal end of the shaft 2d via the wrist joint 2c.

As shown in FIG. 1, the endoscope 3 is attached to the distal end of one of the plurality of robot arms 50, such as the robot arm 50c, and the instrument 2 is attached to the distal end of each of the remaining robot arms 50a, 50b, and 50d, for example. The endoscope 3 is preferably attached to one of two robot arms 50b and 50c arranged in the center among the four robot arms 50 arranged adjacent to each other. The robot arms 50a, 50b, and 50d are examples of a second robot arm, and the robot arm 50c is an example of a first robot arm.

### Configuration of Instrument

As shown in FIG. 5, the end effector 2b including jaw members 2g and 2h is attached to the distal end of the instrument 2, for example. As the end effector 2b, a pair of scissors, a grasper, a needle holder, a microdissector, a stable applier, a tacker, a suction cleaning tool, a snare wire, a clip applier, etc. can be used.

The instrument 2 includes a first support member 2f and a second support member 2e. The second support member 2e is attached to the shaft 2d. The first support member 2f is supported by the second support member 2e so as to be rotatable around an A10 axis, and supports the end effector 2b such that the end effector 2b is rotatable around an A11 axis that intersects with the A10 axis as viewed in a direction in which the shaft 2d extends. The shaft 2d rotates around an A9 axis. The wrist joint 2c is provided between the first support member 2f and the second support member 2e with the A10 axis as a rotation axis. The A10 axis and the A11 axis are examples of a second rotation axis and a first rotation axis, respectively.

### Configuration of Arm Operation Unit

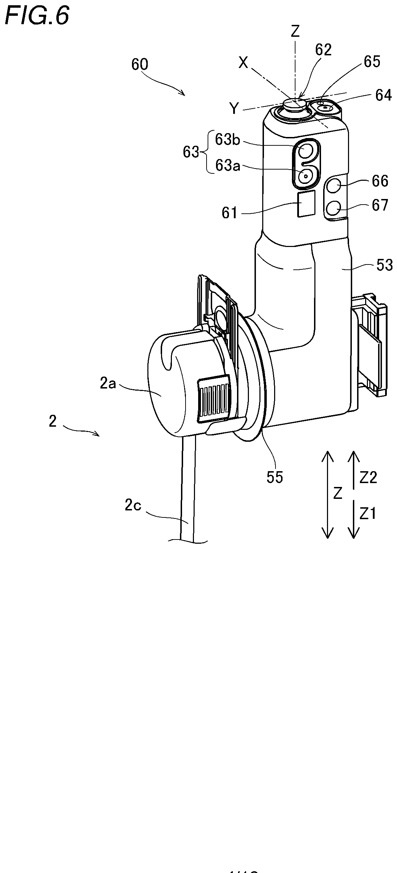

As shown in FIG. 6, the arm operation unit 60 is attached to the robot arm 50 to operate the robot arm 50. Specifically, the arm operation unit 60 is attached to the second link 53.

The arm operation unit 60 includes an enable switch 61, a joystick 62, and linear switches 63, a mode switching button 64, a mode indicator 65, a pivot button 66, and an adjustment button 67.

The enable switch 61 is pressed to enable or disable movement of the robot arm 50 in response to the joystick 62 and the linear switches 63. When the enable switch 61 is pressed by an operator such as a nurse or an assistant grasping the arm operation unit 60, movement of the surgical instrument 1 by the robot arm 50 is enabled.

The joystick 62 is an operation tool to control movement of the surgical instrument 1 by the robot arm 50. The joystick 62 controls a moving direction and a moving speed of the robot arm 50. The robot arm 50 is moved in accordance with a tilting direction and a tilting angle of the joystick 62.

The linear switches 63 are switches to move the surgical instrument 1 in the Z direction, which is the longitudinal direction of the surgical instrument 1. The linear switches 63 include a linear switch 63a to move the surgical instrument 1 in a direction in which the surgical instrument 1 is inserted into a patient P, and a linear switch 63b to move the surgical instrument 1 in a direction in which the surgical instrument 1 is moved away from the patient P. Both the linear switch 63a and the linear switch 63b are push-button switches.

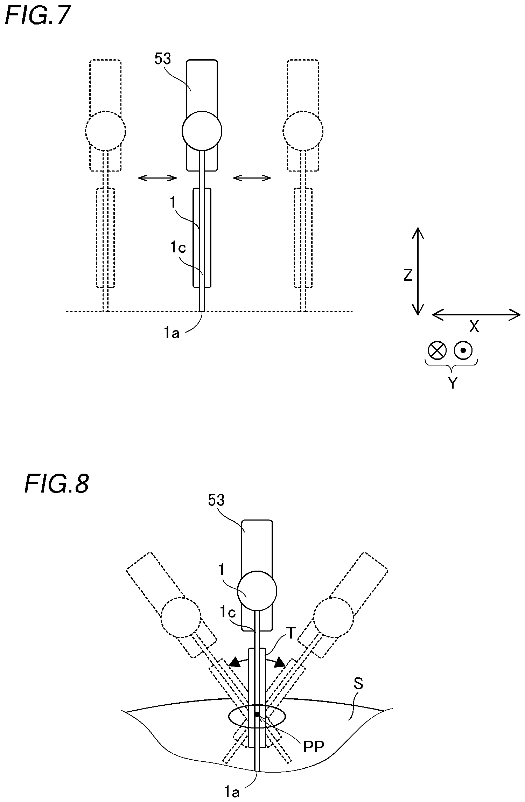

The mode switching button 64 is a push-button switch to switch between a mode for translationally moving the surgical instrument 1 and a mode for rotationally moving the surgical instrument 1. As shown in FIG. 7, in the mode for translationally moving the robot arm 50, the robot arm 50 is moved such that the distal end 1a of the surgical instrument 1 is moved in an X-Y plane. As shown in FIG. 8, in the mode for rotationally moving the robot arm 50, the robot arm 50 is moved such that the surgical instrument 1 is rotationally moved around a center on the A11 axis of the end effector 2b or the distal end of the end effector 2b of the instrument 2 as the surgical instrument 1 as a fulcrum when any pivot position PP is not stored in a storage 351, and the surgical instrument 1 is rotationally moved around the pivot position PP as a fulcrum when the pivot position PP is stored in the storage 351. In this case, the surgical instrument 1 is rotationally moved with the shaft 1c of the surgical instrument 1 inserted into a trocar T. The mode switching button 64 is arranged on a Z-direction side surface of the arm operation unit 60.

The mode indicator 65 indicates a switched mode. The mode indicator 65 is on to indicate a rotational movement mode and is off to indicate a translational movement mode. Furthermore, the mode indicator 65 also serves as a pivot position indicator that indicates that the pivot position PP has been set. The mode indicator 65 is arranged on the Z-direction side surface of the arm operation unit 60.

The pivot button 66 is a push-button switch to set the pivot position PP that serves as a fulcrum for movement of the surgical instrument 1 attached to the robot arm 50.

The adjustment button 67 is a button to optimize the position of the robot arm 50. After the pivot position PP for the robot arm 50 to which the endoscope 3 has been attached is set, the positions of the other robot arms 50 and the arm base 40 are optimized when the adjustment button 67 is pressed. The adjustment button 67 is a button different from the enable switch 61.

### Remote Control Apparatus

As shown in FIG. 1, the remote control apparatus 200 is arranged inside or outside the operating room, for example. The remote control apparatus 200 includes the operation units 110, foot pedals 120, a touch panel 130, a monitor 140, a support arm 150, a support bar 160, an error reset button 161. The operation units 110 include operation handles for the operator such as a doctor to input a command.

### Operation Unit

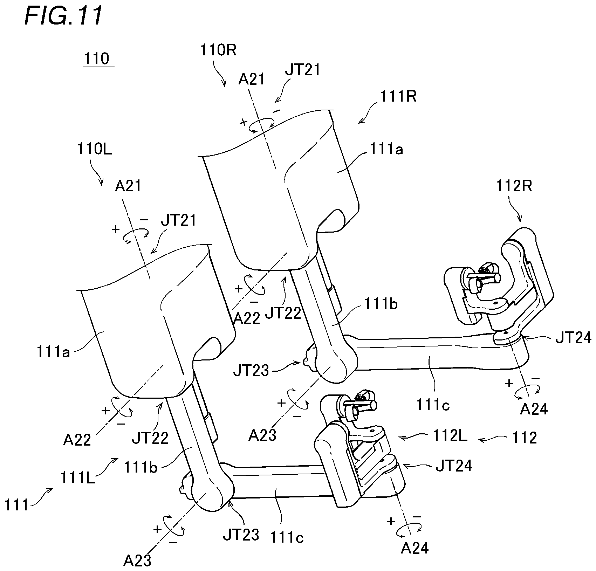

As shown in FIG. 11, the operation units 110 each include a handle to operate the surgical instrument 1. The operation unit 110 receives an operation for the surgical instrument 1. The operation units 110 include an operation unit 110L that is located on the left side as viewed from the operator such as a doctor and is to be operated by the left hand of the operator, and an operation unit 110R that is located on the right side and is to be operated by the right hand of the operator. The operation units 110 include arms 111 and wrists 112. The operation unit 110R includes an arm 111R and a wrist 112R. The operation unit 110L includes an arm 111L and a wrist 112L. The operation unit 110R and the operation unit 110L are examples of a right-handed operation unit and a left-handed operation unit, respectively.

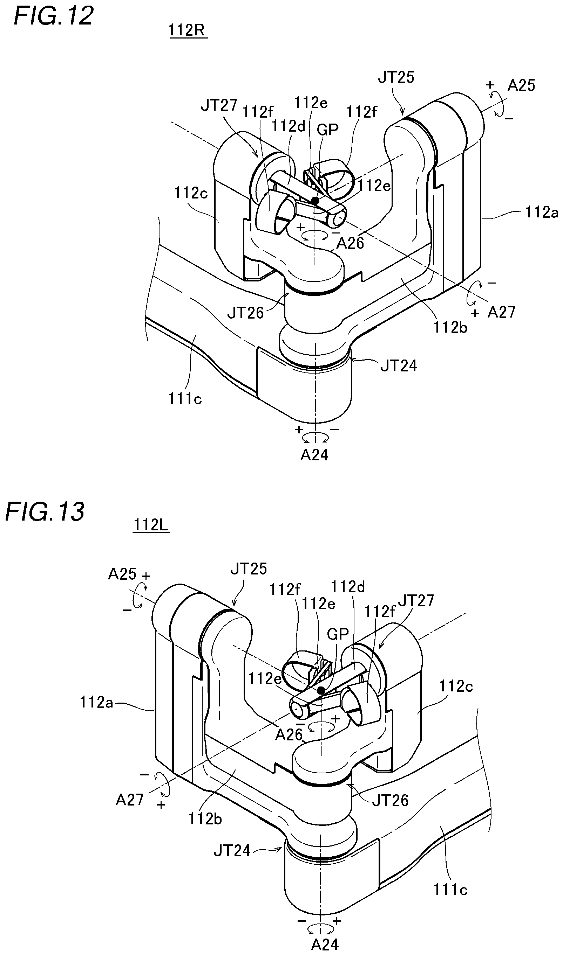

The operation units 110 each have joints JT21, JT22, and JT23 shown in FIG. 11, and joints JT24, JT25, JT26, and JT27 shown in FIGS. 12 and 13. The rotation axes of the joints JT21, JT22, JT23, JT24, JT25, JT26, and JT27 are A21, A22, A23, A24, A25, A26, and A27 axes, respectively.

As shown in FIG. 11, the arm 111R includes a link 111a, a link 111b, and a link 111c. The upper end side of the link 111a is attached to the remote control apparatus 200 such that the link 111a is rotatable around the A21 axis along a vertical direction. The upper end side of the link 111b is attached to the lower end side of the link 111a such that the link 111b is rotatable around the A22 axis along a horizontal direction. A first end side of the link 111c is attached to the lower end side of the link 111b such that the link 111c is rotatable around the A23 axis along the horizontal direction. The wrist 112 is attached to a second end side of the link 111c such that the wrist 112 is rotatable around the A24 axis. The link 111a is connected to the remote control apparatus 200 by the joint JT21. The link 111a and the link 111b are connected to each other by the joint JT22. The link 111b and the link 111c are connected to each other by the joint JT23. The arm 111 supports the wrist 112. The arm 111L has the same or similar configuration as the arm 111R.

The wrists 112 include the wrist 112R shown in FIG. 12 and to be operated by the right hand of the operator, and the wrist 112L shown in FIG. 13 and to be operated by the left hand of the operator. FIG. 12 shows the reference posture of the operation unit 110R, and FIG. 13 shows the reference posture of the operation unit 110L. The configuration of the wrist 112R is the same as or similar to that of the wrist 112L.

The wrists 112 each include a link 112a, a link 112b, a link 112c, and a grip support member 112d that is to be operated by the operator such as a doctor. The link 112a includes a proximal end connected to the distal end of the arm 111 and rotates around the A24 axis. The link 112b includes a proximal end connected to the distal end of the link 112a and rotates around the A25 axis with respect to the link 112a. The link 112c includes a proximal end connected to the distal end of the link 112b and a distal end to which the grip support member 112d is connected, and rotates around the A26 axis with respect to the link 112b. The grip support member 112d rotates around the A27 axis with respect to the link 112c. The link 112a, the link 112b, and the link 112c each have an L shape.

The wrist 112 includes a pair of grip members 112e that are opened and closed by the operator. The grip members 112e each include an elongated plate-shaped lever member, and the proximal ends of the pair of grip members 112e are rotatably connected to the proximal end of the grip support member 112d. Cylindrical finger insertion portions 112f are provided on the grip members 112e. The operator inserts their fingers into a pair of finger insertion portions 112f to operate the wrist 112. The proximal ends of the pair of grip members 112e are connected to the grip support member 112d, and an angle between the pair of grip members 112e is increased or decreased such that an opening angle between the jaw member 2g and the jaw member 2h is changed. A magnet is provided on one of the grip members 112e, and a Hall sensor is provided on the grip support member 112d. When the operator opens and closes the grip members 112e, the magnet and the Hall sensor function as an angle detection sensor, and the Hall sensor outputs the opening angle. As the angle detection sensor, the Hall sensor may be provided on the grip member 112e, and the magnet may be provided on the grip support member 112d. Alternatively, the magnet or the Hall sensor may be provided as the angle detection sensor on both the grip members 112e.

As shown in FIG. 1, the monitor 140 is a scope-type display that displays images captured by the endoscope 3. A notifier 141 is provided on the monitor 140. The notifier 141 issues an error sound. The support arm 150 supports the monitor 140 so as to align the height of the monitor 140 with the height of the face of the operator such as a doctor. The touch panel 130 is arranged on the support bar 160. The head of the operator is detected by a sensor provided in the vicinity of the monitor 140 such that the surgical robot 100 can be operated by the remote control apparatus 200. The operator operates the operation units 110 and the foot pedals 120 while visually recognizing an affected area on the monitor 140. Thus, a command is input to the remote control apparatus 200. The command input to the remote control apparatus 200 is transmitted to the surgical robot 100.

### Foot Pedals

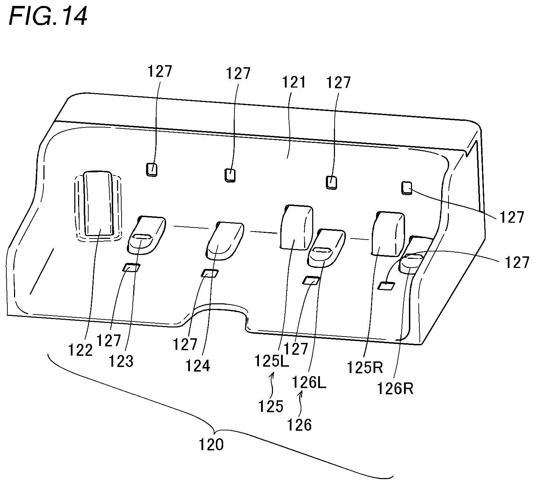

As shown in FIG. 14, a plurality of foot pedals 120 are provided to perform functions related to the surgical instrument 1. The plurality of foot pedals 120 are arranged on a base 121. The foot pedals 120 include a switching pedal 122, a clutch pedal 123, a camera pedal 124, incision pedals 125, coagulation pedals 126, and foot detectors 127. The switching pedal 122, the clutch pedal 123, the camera pedal 124, the incision pedals 125, and the coagulation pedals 126 are operated by the foot of the operator. The incision pedals 125 include an incision pedal 125R for a right robot arm 50, and an incision pedal 125L for a left robot arm 50. The coagulation pedals 126 include a coagulation pedal 126R for the right robot arm 50 and a coagulation pedal 126L for the left robot arm 50.

The switching pedal 122 switches robot arms 50 to be operated by the operation units 110. The clutch pedal 123 performs a clutch operation to temporarily disconnect an operation connection between the robot arms 50 and the operation units 110. While the clutch pedal 123 is being pressed by the operator, operations by the operation units 110 are not transmitted to the robot arms 50. While the camera pedal 124 is being pressed by the operator, the operation unit 110 can operate a robot arm 50 to which the endoscope 3 is attached. While the incision pedals 125 or the coagulation pedals 126 are being pressed by the operator, an electrosurgical device is activated.

### Vision Unit and Image Processing Unit

As shown in FIG. 1, the vision unit 300 and the image processing unit 400 are placed on a cart 210. The image processing unit 400 processes images captured by the endoscope 3. A display 220 is arranged on the cart 210. The display 220 displays images captured by the endoscope 3.

### Configuration of Control System

As shown in FIG. 15, the robotic surgical system 500 includes a first control device 310, an arm controller 320, a positioner controller 330, operation controllers 340, and a second control device 350. The robotic surgical system 500 also includes a storage 311 connected to the first control device 310 and the storage 351 connected to the second control device 350. The first control device 310 is an example of a controller.

The first control device 310 is accommodated in the medical cart 10 to communicate with the arm controller 320 and the positioner controller 330, and controls the entire robotic surgical system 500. Specifically, the first control device 310 communicates with and controls the arm controller 320, the positioner controller 330, and the operation controllers 340. The first control device 310 is connected to the arm controller 320, the positioner controller 330, and the operation controllers 340 through a LAN, for example. The first control device 310 is arranged inside the medical cart 10.

The arm controller 320 is arranged for each of the plurality of robot arms 50. That is, the same number of arm controllers 320 as the plurality of robot arms 50 are placed inside the medical cart 10.

As shown in FIG. 15, the input 22 is connected to the first control device 310 through a LAN, for example. The status indicator 41, the arm status indicator 42, the operation handle 23, the throttle 23a, and the joystick 22b are connected to the positioner controller 330 via a wire line 360 by means of a communication network that allows information to be shared with each other by using serial communication. Although FIG. 15 shows that the status indicator 41, the arm status indicator 42, etc. are all connected to one wire line 360, in reality, the wire line 360 is arranged for each of the status indicator 41, the arm status indicator 42, the operation handle 23, the throttle 23a, the joystick 22b, the stabilizer 24, and the electric cylinder 25.

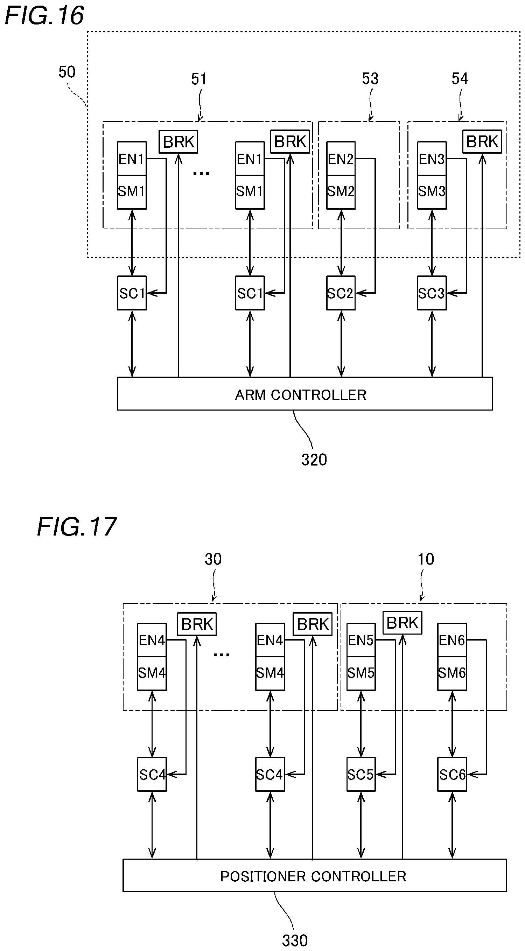

As shown in FIG. 16, the arm portion 51 includes a plurality of servomotors SM1, encoders EN1, and speed reducers so as to correspond to the joints JT1, JT2, JT3, JT4, JT5, JT6, and JT7. The encoders EN1 detect rotation angles of the servomotors SM1. The speed reducers slow down rotation of the servomotors SM1 to increase the torques. Inside the medical cart 10, servo controllers SC1 that control the servomotors SM1 are arranged adjacent to the arm controller 320. In addition, the encoders EN1 that detect the rotation angles of the servomotors SM1 are electrically connected to the servo controllers SC1.

Servomotors SM2 that rotate driven members provided in the driven unit 2a of the surgical instrument 1, encoders EN2, and speed reducers are arranged in the second link 53. The encoders EN2 detect rotation angles of the servomotors SM2. The speed reducers slow down rotation of the servomotors SM2 to increase the torques. In the medical cart 10, servo controllers SC2 are provided to control the servomotors SM2 to drive the surgical instrument 1. The encoders EN2 that detect the rotation angles of the servomotors SM2 are electrically connected to the servo controllers SC2. A plurality of servomotors SM2, a plurality of encoders EN2, and a plurality of servo controllers SC2 are arranged.

The translation mechanism 54 includes a servomotor SM3 to translationally move the surgical instrument 1, an encoder EN3, and a speed reducer. The encoder EN3 detects a rotation angle of the servomotor SM3. The speed reducer slows down rotation of the servomotor SM3 to increase the torque. In the medical cart 10, a servo controller SC3 is provided to control the servomotor SM3 to translationally move the surgical instrument 1. The encoder EN3 that detects the rotation angle of the servomotor SM3 is electrically connected to the servo controller SC3.

The first control device 310 generates command values to command the positions of the servomotors SM1, SM2, and SM3 based on operations received by the remote control apparatus 200, and drives the servomotors SM1, SM2, and SM3 based on the command values. The first control device 310 detects an abnormal deviation error when differences between the command values and the positions of the servomotors SM1, SM2, and SM3 detected by sensors exceed an allowable range.

As shown in FIG. 17, a plurality of servomotors SM4, a plurality of encoders EN4, and a plurality of speed reducers are provided in the positioner 30 so as to correspond to the plurality of joints 33 of the positioner 30. The encoders EN4 detect rotation angles of the servomotors SM4. The speed reducers slow down rotation of the servomotors SM4 to increase the torques.

The medical cart 10 includes wheels including front wheels as drive wheels and rear wheels that are steered by the operation handle 23. The rear wheels are arranged closer to the operation handle 23 than the front wheels. The medical cart 10 includes servomotors SM5 to drive a plurality of front wheels of the medical cart 10, encoders EN5, speed reducers, and brakes BRK. The speed reducers slow down rotation of the servomotors SM5 to increase the torques. A potentiometer P1 shown in FIG. 3 is provided on the operation handle 23, and the servomotors SM5 of the front wheels are driven based on a rotation angle detected by the potentiometer P1 according to the twist of the throttle 23a. Rear wheels of the medical cart 10 are of the dual wheel type, and the rear wheels are steered based on rightward-leftward rotation of the operation handle 23. Furthermore, a potentiometer P2 shown in FIG. 3 is provided on a rotation axis of the operation handle 23, and servomotors SM6, encoders EN6, and speed reducers are provided on the rear wheels of the medical cart 10. The speed reducers slow down rotation of the servomotors SM6 to increase the torques. The servomotors SM6 are driven based on a rotation angle detected by the potentiometer P2 according to rightward-leftward rotation of the operation handle 23. That is, steering of the rear wheels by the rightward-leftward rotation of the operation handle 23 is power-assisted by the servomotors SM6.

The front wheels of the medical cart 10 are driven such that the medical cart 10 moves in the forward-rearward direction. Furthermore, the operation handle 23 is rotated such that the rear wheels are steered, and the medical cart 10 turns in a right-left direction.

As shown in FIG. 17, in the medical cart 10, servo controllers SC4 are provided to control the servomotors SM4 to move the positioner 30. The encoders EN4 that detect the rotation angles of the servomotors SM4 are electrically connected to the servo controllers SC4. In the medical cart 10, servo controllers SC5 are provided to control the servomotors SM5 to drive the front wheels of the medical cart 10. The encoders EN5 that detect the rotation angles of the servomotors SM5 are electrically connected to the servo controllers SC5. In the medical cart 10, servo controllers SC6 are provided to control the servomotors SM6 to power-assist steering of the rear wheels of the medical cart 10. The encoders EN6 that detect the rotation angles of the servomotors SM6 are electrically connected to the servo controllers SC6.

As shown in FIGS. 16 and 17, the joints JT1, JT2, JT3, JT4, JT5, JT6, and JT7 of the arm portion 51, and the joints 33 of the positioner 30 include brakes BRK. Furthermore, the front wheels of the medical cart 10, the arm base 40, and the translation mechanism 54 include brakes BRK. The arm controller 320 unidirectionally transmits control signals to the brakes BRK of the joints JT1, JT2, JT3, JT4, JT5, JT6, and JT7 of the arm portion 51, and the translation mechanism 54. The control signals are signals for turning on/off the brakes BRK. The signals for turning on the brakes BRK include signals for maintaining the brakes BRK in an enabled state. The same applies to control signals from the positioner controller 330 to the brakes BRK of the joints 33 of the positioner 30 and the arm base 40. On startup, all the brakes BRK of the arm base 40, the arm portion 51, and the translation mechanism 54 are turned off but the servomotors SM are driven against gravity to maintain the postures of the robot arm 50 and the arm base 40. When an error occurs in the robotic surgical system 500, the brakes BRK of the arm base 40, the arm portion 51 and the translation mechanism 54 are turned on. When the error in the robotic surgical system 500 is reset, the brakes BRK of the arm base 40, the arm portion 51, and the translation mechanism 54 are turned off. When a shutdown operation is performed in the robotic surgical system 500, the brakes BRK of the arm base 40, the arm portion 51, and the translation mechanism 54 are turned on. The brakes BRK of the front wheels of the medical cart 10 are constantly turned on, and the brakes BRK are deactivated only while the enable switch 23b is being pressed. The brakes BRK of the joints 33 of the positioner 30 are constantly turned on, and the brakes BRK are deactivated only while the enable switch 22c is being pressed.

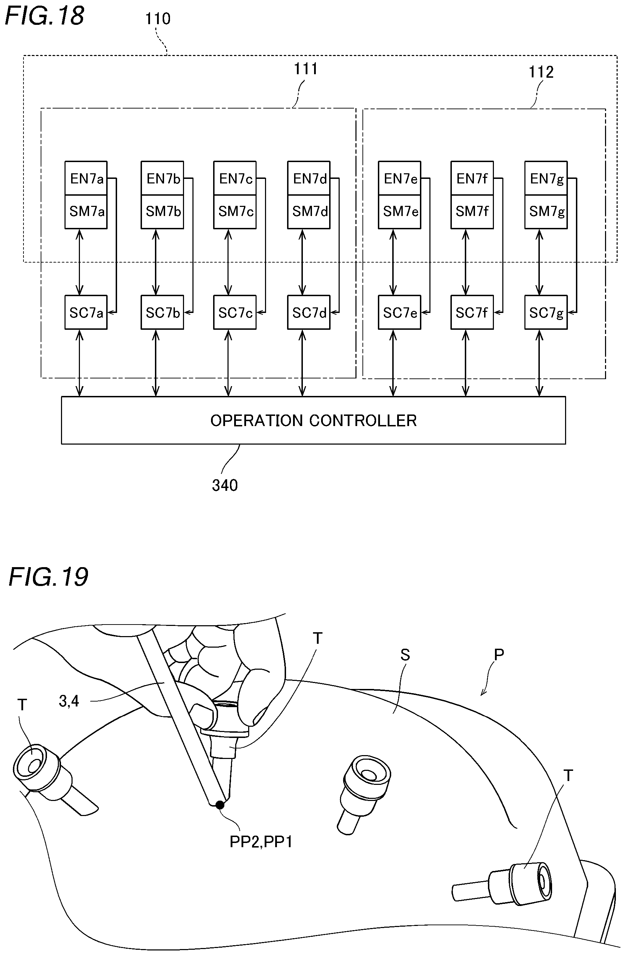

As shown in FIG. 18, servomotors SM7a, SM7b, SM7c, SM7d, SM7e, SM7f, and SM7g are arranged on the joints JT21, JT22, JT23, JT24, JT25, JT26, and JT27 of the operation unit 110, respectively. The servomotor SM7a rotates the link 111a around the A21 axis. The servomotor SM7b rotates the link 111b around the A22 axis. The servomotor SM7c rotates the link 111c around the A23 axis. The servomotor SM7d rotates the link 112a around the A24 axis. The servomotor SM7e rotates the link 112b around the A25 axis. The servomotor SM7f rotates the link 112c around the A26 axis. The servomotor SM7g rotates the grip support member 112d around the A27 axis. Servo controllers SC7a, SC7b, SC7c, SC7d, SC7e, SC7f, and SC7g are provided to control the servomotors. Encoders EN7a, EN7b, EN7c, EN7d, EN7e, EN7f, and EN7g are electrically connected to the servo controllers to detect rotation angles of the servomotors. The servomotors, the servo controllers, and the encoders are provided in each of the operation unit 110L and the operation unit 110R.

The first control device 310 controls the servomotors via the operation controllers 340 to generate torques that counteract gravitational torques generated on rotation axes of the servomotors according to the postures of the operation units 110. Thus, the operator can operate the operation units 110 with a relatively small force.

When the operator performs an operation to rotate the grip support member 112d around the A27 axis of the operation unit 110 shown in FIGS. 12 and 13, the shaft 2d of the instrument 2 rotates around the A9 axis shown in FIG. 5. When the operator performs an operation to rotate the joints JT24, JT25, and JT26 of the operation unit 110 shown in FIGS. 12 and 13, the end effector 2b bends around the A10 axis or the A11 axis shown in FIG. 5.

The operation controllers 340 are arranged in a main body of the remote control apparatus 200. The operation controllers 340 control the operation units 110. The operation controllers 340 are provided so as to correspond to the left-handed operation unit 110L and the right-handed operation unit 110R, respectively, as shown in FIG. 15.

As shown in FIG. 15, the vision unit 300 and the image processing unit 400 are connected to the first control device 310 through a LAN, for example. The display 220 is connected to the vision unit 300.

### Pivot Position Setting

Setting of the pivot position PP is now described. As shown in FIG. 19, when the pivot button 66 is operated in a state in which the distal end of the endoscope 3 attached to the distal end side of the robot arm 50 has been moved to a position corresponding to the insertion position of the trocar T inserted into the body surface S of the patient P by operating the robot arm 50 with the arm operation unit 60, the second control device 350 causes the storage 351 to store the pivot position PP2 of the endoscope 3. Similarly, when the pivot button 66 is operated in a state in which the distal end of the pivot position setting instrument 4 attached to the distal end side of the robot arm 50 has been moved to the position corresponding to the insertion position of the trocar T inserted into the body surface S of the patient S, the second control device 350 causes the storage 351 to store the pivot position PP1 of the instrument 2. Operating the pivot button 66 refers to pressing the pivot button 66. The pivot positions PP1 and PP2 are collectively referred to as pivot positions PP.

### Control Operation of First Control Device

A control performed by the first control device 310 when the operation unit 110 receives an operation by the operator is now described.

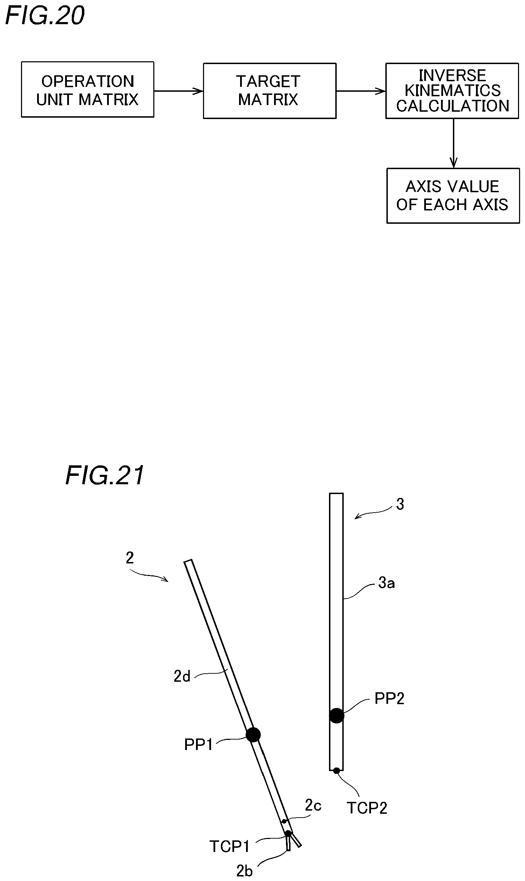

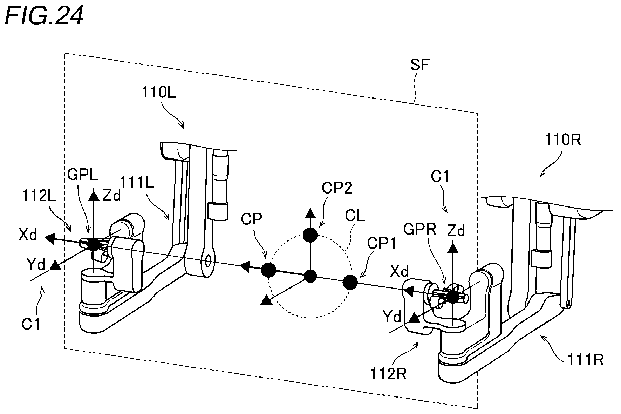

As shown in FIG. 20, the first control device 310 presets an operation unit matrix for the operation unit 110. The operation unit matrix is a homogeneous transformation matrix of a 4 × 4 matrix. The operation unit matrix represents the result of forward kinematics calculation for the position and posture of the operation unit 110. The operation unit matrix is set based on the coordinate system C1 of the operation unit 110 shown in FIG. 24. Next, the operation by the operator is received by the operation unit 110. Thus, a target matrix that is a target corresponding to the received operation is generated. The target matrix is also a homogeneous transformation matrix. The target matrix represents target values of the position and posture of the surgical instrument 1. The target matrix is set based on the coordinate system of the surgical robot 100. The coordinate system of the surgical robot 100 is an endoscope coordinate system, for example. The homogeneous transformation matrix includes a translation component for translation of the surgical instrument 1 and a rotation component for rotation of the surgical instrument 1. Specifically, the first control device 310 calculates a difference between the current position of the surgical instrument 1 and a target position received by the operation unit 110. The position corresponds to the translation component of the homogeneous transformation matrix. The first control device 310 calculates a difference between the current posture of the surgical instrument 1 and a target posture received by the operation unit 110. The posture corresponds to the rotation component of the homogeneous transformation matrix. The first control device 310 calculates the target matrix based on the calculated difference value. Next, the first control device 310 performs an inverse kinematics calculation on the updated homogeneous transformation matrix. The first control device 310 calculates axis values of joint axes and linear motion axes for the robot arm 50 and the surgical instrument 1 by the inverse kinematics calculation. Thus, the positions and postures of the robot arm 50 and the surgical instrument 1 are changed to conform to the received operation.

### Control Operation of First Control Device for Instrument

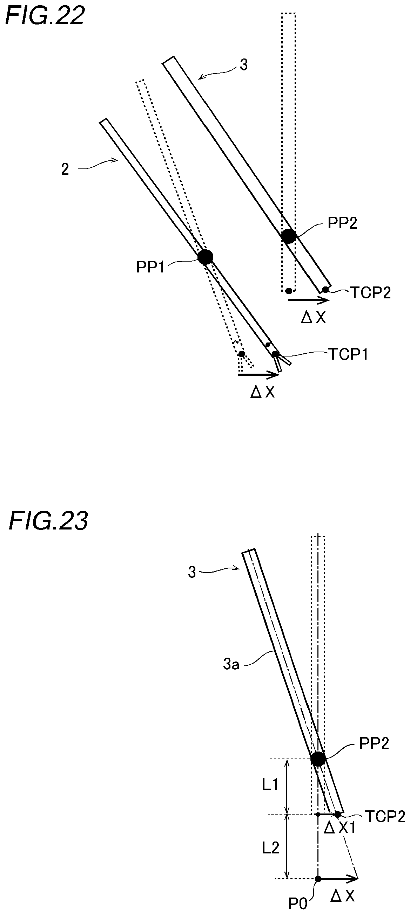

First, as shown in FIG. 21, the end effector 2b at the distal end of the instrument 2 is assumed to be located within the field of view of the endoscope 3. Next, as shown in FIG. 22, in this embodiment, when the operation unit 110 receives an operation to move the instrument 2 by a given distance ΔX, the first control device 310 performs a process to move a control point set at the distal end of the instrument 2 by a distance corresponding to the given distance ΔX. Specifically, when the operation unit 110 receives an operation to move the instrument 2 by the given distance ΔX, the first control device 310 performs a process to move the control point set at the distal end of the instrument 2 by the distance corresponding to the given distance ΔX with the pivot position PP1, which serves as a fulcrum for movement of the instrument 2, as the center. The distal end of the instrument 2 refers to a portion between the wrist joint 2c of the instrument 2 and the distal end of the end effector 2b. In other words, the distal end of the instrument 2 refers to a portion between the A10 axis and the distal end of the end effector 2b. The distance corresponding to the given distance ΔX refers to a distance obtained by scaling the given distance ΔX described below. FIG. 22 shows a case in which the magnitude of the scaling is 1 and the given distance ΔX = the distance corresponding to the given distance ΔX. More specifically, when the operation unit 110 receives an operation to move the instrument 2 by the given distance ΔX, the first control device 310 performs a process to move a tool center point TCP1 set on the rotation axis of the end effector 2b by the given distance ΔX with the pivot position PP1 as the fulcrum. The tool center point is a control point that is the target of the operation control of the instrument 2 or the endoscope 3, and can be set at any position inside or on a surface of the instrument 2 or the endoscope 3. For example, in the instrument 2, the control point is set to the distal end of the end effector 2b, the intersection of the longitudinal axis of the shaft 2d with the A10 axis, or the intersection of the longitudinal axis of the shaft 2d with the A11 axis, but is not limited to this. In FIG. 5, the tool center point TCP1 is set to the intersection of the longitudinal axis of the shaft 2d with the A11 axis. When the operation unit 110 receives an operation to move the instrument 2, the first control device 310 performs a process to scale the received operation amount and actually move the instrument 2. Scaling indicates that the movement amount of the instrument 2 becomes smaller than the movement amount of the operation unit 110 moved by the operator. For example, the movement amount of the instrument 2 is set to be one-third of the movement amount of the operation unit 110 moved by the operator. The magnitude of the scaling may be a fixed value or may be variable by the operator. The magnitude of the scaling is between 1.5:1 and 3:1, for example. The scaling is performed when the target matrix is generated from the operation unit matrix described above.

In this embodiment, an operation to move the instrument 2 is received by one of the operation units 110R and 110L. As described above, the instrument 2 is attached to the robot arms 50a, 50b, and 50d, for example. As described above, the robot arm 50 to be operated by the operation unit 110 is switched by the switching pedal 122 of the foot pedals 120. Thus, the robot arm 50 to be operated by each of the operation units 110R and 110L is switched. Then, an operation for one robot arm 50 is received by one of the operation units 110R and 110L, and the instrument 2 attached to that robot arm 50 is moved.

### Control Operation of First Control Device for Endoscope

As shown in FIG. 22, when the operation unit 110 receives an operation to move the endoscope 3 by the given distance ΔX, the distal end of the endoscope 3 moves relatively largely when a tool center point TCP2 set at the distal end of the endoscope 3 is moved by the distance corresponding to the given distance ΔX. Therefore, the end effector 2b at the distal end of the instrument 2 is outside the field of view of the endoscope 3. Therefore, in this embodiment, as shown in FIG. 23, when the operation unit 110 receives an operation to move the endoscope 3 by the given distance ΔX, the first control device 310 performs a process to move a control point set at a virtual position P0 offset ahead of the distal end of the endoscope 3 in the axial direction of the endoscope 3 by the distance corresponding to the given distance ΔX. In this embodiment, the robot arm 50c can rotate the endoscope 3 around the longitudinal axis of the endoscope 3, and the control point set at the virtual position P0 is set on the longitudinal axis of the endoscope 3. The virtual position P0 is a position away from the distal end of the endoscope 3. Furthermore, when the operation unit 110 receives an operation to move the endoscope 3 by the given distance ΔX, the first control device 310 performs a process to move the control point set at the virtual position P0 by the distance corresponding to the given distance ΔX with pivot position PP2 that serves as a fulcrum for movement of the endoscope 3 as the center. The distance corresponding to the given distance ΔX refers to a distance obtained by scaling the given distance ΔX described below. FIG. 23 shows a case in which the magnitude of the scaling is 1 and the given distance ΔX = the distance corresponding to the given distance ΔX. Specifically, when the operation unit 110 receives an operation to move the endoscope 3 by the given distance ΔX, the first control device 310 performs a process to move the tool center point TCP2 set in the endoscope 3 by the given distance ΔX with the pivot position PP2 as the fulcrum. The tool center point TCP2 of the endoscope 3 is set to, for example, the intersection of the axis of the shaft 3a of the endoscope 3 with the end face of the endoscope 3, but is not limited to this.

In this embodiment, as shown in FIG. 23, when the operation unit 110 receives an operation to move the endoscope 3 by the given distance ΔX, the first control device 310 performs a process to move the control point set at the virtual position P0 by a distance ΔX1 corresponding to the given distance ΔX based on a relational expression ΔX1 = ΔX × L1/(L1 + L2), where ΔX represents the given distance ΔX, ΔX1 represents the distance corresponding to the given distance ΔX, L1 represents a distance from the pivot position PP2 to the distal end of the endoscope 3, and L2 represents a distance from the distal end of the endoscope 3 to the virtual position P0. That is, the distal end of the endoscope 3 moves the distance ΔX1 smaller than the distance ΔX based on the relational expression. The relational expression is used when the target matrix is generated from the operation unit matrix described above. The virtual position P0 is a fixed position. That is, the distance L2 from the distal end of the endoscope 3 to the virtual position P0 is a fixed value. Furthermore, L2 is, for example, 20 mm or more and 80 mm or less, and preferably 50 mm. The virtual position P0 is a position determined based on a distance from the distal end of the endoscope 3 to the distal end of the instrument 2, and the virtual position P0 is set as the control point of the endoscope 3 such that a difference in the movement amount between the endoscope 3 and the instrument 2 perceived by the operator can be reduced.

In this embodiment, when the operation unit 110 receives an operation to move the endoscope 3, the first control device 310 performs a process to scale the received operation amount by the same amount as when the instrument 2 is moved and actually move the endoscope 3. For example, as described above, when the movement amount of the instrument 2 is set to be one-third of the movement amount of the operation unit 110 moved by the operator, the movement amount of the endoscope 3 is also set to be one-third of the movement amount of the operation unit 110 moved by the operator. In such a case, the distance ΔX1 described above becomes a distance ΔX1/3. The magnitude of the scaling of the endoscope 3 may also be a fixed value or may be variable by the operator. The scaling is also performed when the target matrix is generated from the operation unit matrix described above.

In this embodiment, the operation to move the endoscope 3 is received by both the operation unit 110R and the operation unit 110L. Specifically, while the camera pedal 124 of the foot pedals 120 shown in FIG. 14 is being pressed by the operator, the operation unit 110 can operate the robot arm 50 to which the endoscope 3 is attached. More specifically, as shown in FIG. 24, the first control device 310 defines a virtual plane SF based on the coordinate system C1 of the operation unit 110. A method for setting the virtual plane SF is as follows. The gimbal points GP of the operation units 110R and 110L are set as GPR and GPL, respectively. The gimbal points GP are points at which the A25 axis, the A26 axis, and the A27 axis shown in FIGS. 12 and 13 intersect with each other. The midpoint of a line segment connecting the gimbal point GPR of the operation unit 110R to the gimbal point GPL of the operation unit 110L is set as CP. A point, the Y coordinate of which is the same as that of the midpoint CP and the X and Z coordinates of which are the same as those of a midpoint between the midpoint CP and the gimbal point GPR, is set as CP1. A straight line connecting the midpoint CP to the point CP1 is set as a diameter, and the intersection of a circle CL on a plane that passes through the same Y coordinate as the midpoint CP and the point CP1 with a line perpendicular to the straight line connecting the midpoint CP to the point CP1 is set as CP2. The virtual plane SF is a plane including CP, CP1, and CP2. The first control device 310 sets the movement amount of the virtual plane SF in the previous control cycle and the current control cycle as the movement amount of the distal end of the endoscope 3.

### Control Method for Robotic Surgical System

A control method for the robotic surgical system 500 is now described.

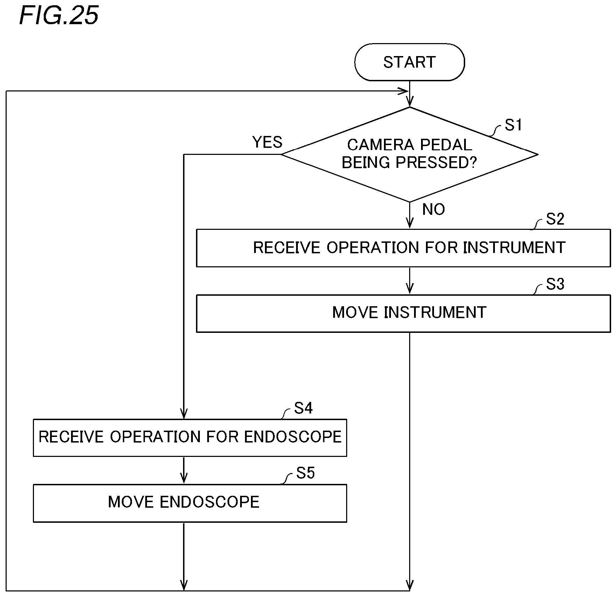

As shown in FIG. 25, in step S1, the first control device 310 determines whether or not the camera pedal 124 of the foot pedals 120 is being pressed by the operator. In a case of no in step S1, in step S2, the operation unit 110 receives an operation for the instrument 2 supported by the robot arm 50. In step S3, when the operation unit 110 receives an operation to move the instrument 2 by the given distance ΔX, the first control device 310 moves the control point set at the distal end of the instrument 2 by the distance corresponding to the given distance ΔX.

In a case of yes in step S1, in step S4, the operation unit 110 receives an operation for the endoscope 3 supported by the robot arm 50. In step S5, when the operation unit 110 receives an operation to move the endoscope 3 by the given distance ΔX, the first control device 310 moves the virtual position P0 offset ahead of the distal end of the endoscope 3 in the axial direction of the endoscope 3 by the distance corresponding to the given distance ΔX. The operations from step S1 to step S5 are constantly performed while the robotic surgical system 500 is in a state in which the same can receive an operation with the operation unit 110.

### Advantages of This Embodiment

The first control device 310 is configured or programmed to perform the process to move the control point set at the virtual position P0 offset ahead of the distal end of the endoscope 3 in the axial direction of the endoscope 3 by the distance corresponding to the given distance ΔX when the operation unit 110 receives an operation to move the endoscope 3 by the given distance ΔX. Accordingly, for example, when the distal end of the endoscope 3 is moved in an arcuate shape with a reference point as a fulcrum, the movement amount of the distal end of the endoscope 3 is smaller when the control point set at the virtual position P0 offset ahead of the distal end of the endoscope 3 is moved by the distance corresponding to the given distance ΔX than when the distal end of the endoscope 3 is moved by the distance corresponding to the given distance ΔX. Consequently, it is possible to reduce or prevent large movement of the field of view of the endoscope 3.

The first control device 310 is configured or programmed to perform the process to move the control point set at the virtual position P0 by the distance corresponding to the given distance ΔX with the pivot position PP2, which serves as a fulcrum for movement of the endoscope 3, as a fulcrum when the operation unit 110 receives an operation to move the endoscope 3 by the given distance ΔX. Accordingly, the movement amount of the distal end of the endoscope 3 is smaller when the virtual position P0 offset ahead of the distal end of the endoscope 3 is moved by the distance corresponding to the given distance ΔX with the pivot position PP2 as the fulcrum than when the distal end of the endoscope 3 is moved by the distance corresponding to the given distance ΔX with the pivot position PP2 as the fulcrum. Consequently, even when the endoscope 3 moves with the pivot position PP2 as the fulcrum, large movement of the field of view of the endoscope 3 can be reduced or prevented.

The robot arm 50c is configured to rotate the endoscope 3 around the longitudinal axis of the endoscope 3, and the control point set at the virtual position P0 is set on the longitudinal axis. Accordingly, the control point is easily set, unlike a case in which the control point set at the virtual position P0 is set at a position deviated from the longitudinal axis.

The first control device 310 is configured or programmed to perform the process to move the control point set at the virtual position P0 by the distance ΔX1 corresponding to the given distance ΔX based on the relational expression ΔX1 = ΔX × L1/(L1 + L2), where ΔX represents the given distance, ΔX1 represents the distance corresponding to the given distance ΔX, L1 represents the distance from the pivot position PP2 to the distal end of the endoscope 3, and L2 represents the distance from the distal end of the endoscope 3 to the virtual position P0, when the operation unit 110 receives an operation to move the endoscope 3 by the given distance ΔX. Accordingly, the first control device 310 can easily calculate the distance ΔX1 corresponding to the given distance ΔX based on the relational equation, and thus the virtual position P0 offset ahead of the distal end of the endoscope 3 can be easily moved.

The first control device 310 is configured or programmed to perform the process to move the control point set at the distal end of the instrument 2 by the distance corresponding to the given distance ΔX when the operation unit 110 receives an operation to move the instrument 2 by the given distance ΔX. Accordingly, even when the operation unit 110 receives operations to move the endoscope 3 and the instrument 2 by the same distance, the movement distance of the distal end of the endoscope 3 is smaller than the movement distance of the control point set at the distal end of the instrument 2, and thus it is possible to reduce or prevent the possibility that the instrument 2 is out of the field of view of the endoscope 3 due to the distal end of the endoscope 3 moving in the same manner as the control point set at the distal end of the instrument 2.

The first control device 310 is configured or programmed to perform the process to move the control point set at the distal end of the instrument 2 by the distance corresponding to the given distance ΔX with the pivot position PP1, which serves as a fulcrum for movement of the instrument 2, as a fulcrum when the operation unit 110 receives an operation to move the instrument 2 by the given distance ΔX. Accordingly, even when the instrument 2 moves with the pivot position PP1 as a fulcrum, the possibility that the instrument 2 is out of the field of view of the endoscope 3 can be reduced or prevented.

The instrument 2 includes the end effector 2b, the first support member 2f to support the end effector 2b such that the end effector 2b is rotatable around the A11 axis, the second support member 2e to support the first support member 2f such that the first support member 2f is rotatable around the A10 axis, and the shaft 2d to support the second support member 2e. The control point is set at the distal end of the end effector 2b, the intersection of the longitudinal axis of the shaft 2d with the A11 axis, or the intersection of the longitudinal axis of the shaft 2d with the A10 axis. Accordingly, when the operation unit 110 receives an operation to move the instrument 2 by the given distance ΔX, the vicinity of the distal end of the end effector 2b moves the distance corresponding to the given distance ΔX, and thus the operator can operate the instrument 2 without feeling any discomfort.

The first control device 310 is configured or programmed to perform the process to scale the received operation amount and actually move the instrument 2 when the operation unit 110 receives an operation to move the instrument 2, and perform the process to scale the received operation amount by the same amount as when the instrument 2 is moved and actually move the endoscope 3 when the operation unit 110 receives an operation to move the endoscope 3. When the instrument 2 and the endoscope 3 are moved in the forward-rearward direction with respect to the operator, the instrument 2 moves in a direction of view of the endoscope 3 even when the magnitude of the scaling is different between the instrument 2 and the endoscope 3, and thus the instrument 2 does not fall out of the field of view of the endoscope 3. On the other hand, when the instrument 2 and the endoscope 3 are moved in the right-left direction or an upward-downward direction with respect to the operator, the instrument 2 may fall out of the field of view of the endoscope 3 when the magnitude of the scaling is different between the instrument 2 and the endoscope 3. Therefore, due to the same magnitude of the scaling between the instrument 2 and the endoscope 3, the possibility that the instrument 2 is out of the field of view of the endoscope 3 can be reduced or prevented.

The operation unit 110 includes the operation unit 110R to be operated by the right hand of the operator and the operation unit 110L to be operated by the left hand of the operator. An operation to move the instrument 2 is received by one of the operation units 110R and 110L, and an operation to move the endoscope 3 is received by both the operation units 110R and 110L. Accordingly, the operation to move the instrument 2 and the operation to move the endoscope 3 are different, and thus the possibility that the operator confuses and operates the instrument 2 and the endoscope 3 can be reduced or prevented.

### Modified Examples

The embodiment disclosed this time must be considered as illustrative in all points and not restrictive. The scope of the present disclosure is not shown by the above description of the embodiment but by the scope of claims for patent, and all modifications or modified examples within the meaning and scope equivalent to the scope of claims for patent are further included.

While the instrument 2 moves with the pivot position PP1 set by pressing the pivot button 66 as a fulcrum, and the endoscope 3 moves with the pivot position PP2 set by pressing the pivot button 66 as a fulcrum in the aforementioned embodiment, the present disclosure is not limited to this. For example, the instrument 2 and the endoscope 3 may alternatively move with a pivot point mechanically determined in advance from the structures of the instrument 2 and the endoscope 3 as a fulcrum.

While the tool center point TCP1 of the instrument 2 is set on the A11 axis, which is the rotation axis of the end effector 2b, in the aforementioned embodiment, the present disclosure is not limited to this. For example, the tool center point TCP1 of the instrument 2 may alternatively be set at the distal end of the end effector 2b. Furthermore, while the tool center point TCP2 of the endoscope 3 is set at the distal end of the endoscope 3 in the aforementioned embodiment, the present disclosure is not limited to this. For example, the tool center point TCP2 of the endoscope 3 may alternatively be set at a position closer to the proximal end than the distal end of the endoscope 3.

While the distal end of the endoscope 3 is moved based on the relational expression ΔX1 = ΔX × L1/(L1 + L2) in the aforementioned embodiment, the present disclosure is not limited to this. For example, the distal end of the endoscope 3 may alternatively be moved based on a relational expression other than the above relational expression or a table in which ΔX1 is associated with ΔX.

While the received operation amount is scaled and the instrument 2 or the endoscope 3 is actually moved when an operation to move the instrument 2 or the endoscope 3 is received in the aforementioned embodiment, the present disclosure is not limited to this. For example, when an operation to move the instrument 2 or the endoscope 3 is received, the instrument 2 or the endoscope 3 may alternatively be moved by a distance equal to the received operation amount without scaling the received operation amount.

While the virtual position P0 is a fixed position, and the distance L2 from the distal end of the endoscope 3 to the virtual position P0 is a fixed value in the aforementioned embodiment, the present disclosure is not limited to this. For example, the distance L2 of the virtual position P0 from the distal end of the endoscope 3 may alternatively be set based on a distance between the control point set at the distal end of the instrument 2 and the distal end of the endoscope 3. For example, the distance L2 may alternatively be set to be greater as the distance between the control point set at the distal end of the instrument 2 and the distal end of the endoscope 3 increases. Thus, the distance L2 is set based on the distance between the control point set at the distal end of the instrument 2 and the distal end of the endoscope 3 such that the distance of the virtual position P0 from the distal end of the endoscope 3 can be set according to the distance between the control point set at the distal end of the instrument 2 and the distal end of the endoscope 3, and thus the possibility that the instrument 2 is out of the field of view of the endoscope 3 can be reduced or prevented regardless of the distance between the control point set at the distal end of the instrument 2 and the distal end of the endoscope 3.

While an operation to move the instrument 2 is received by one of the operation unit 110R and the operation unit 110L in the aforementioned embodiment, the present disclosure is not limited to this. For example, the operation to move the instrument 2 may alternatively be received by both the operation unit 110R and the operation unit 110L. Furthermore, while an operation to move the endoscope 3 is received by both the operation unit 110R and the operation unit 110L in the aforementioned embodiment, the present disclosure is not limited to this. For example, the operation to move the endoscope 3 may alternatively be received by one of the operation unit 110R and the operation unit 110L.

While as the controller according to the present disclosure, the first control device 310 arranged in the medical cart 10 is applied in the aforementioned embodiment, the present disclosure is not limited to this. In the present disclosure, a controller other than the first control device 310 may alternatively be applied as the controller according to the present disclosure.

While four robot arms 50 are provided in the aforementioned embodiment, the present disclosure is not limited to this. In the present disclosure, the number of robot arms 50 may alternatively be other than four.

While each of the arm portion 51 and the positioner 30 includes a 7-axis articulated robot in the aforementioned embodiment, the present disclosure is not limited to this. For example, each of the arm portion 51 and the positioner 30 may alternatively include an articulated robot having an axis configuration other than the 7-axis articulated robot. The axis configuration other than the 7-axis articulated robot refers to six axes or eight axes, for example.

While the surgical robot 100 includes the medical cart 10, the positioner 30, the arm base 40, and the robot arms 50 in the aforementioned embodiment, the present disclosure is not limited to this. For example, the surgical robot 100 may not include the medical cart 10, the positioner 30, or the arm base 40, but may include only the robot arms 50.

While the first control device 310 performs the process to move the tool center point TCP2, which is the control point set at the virtual position P0 offset ahead of the distal end of the endoscope 3 in the axial direction of the endoscope 3, by the distance corresponding to the given distance ΔX when the operation unit 110 receives an operation to move the endoscope 3 by the given distance ΔX in the aforementioned embodiment, the present disclosure is not limited to this. For example, the tool center point TCP2 may alternatively be set at the distal end of the endoscope 3, and the tool center point TCP2 may alternatively be moved such that the virtual position P0 offset from the tool center point TCP2 moves the distance corresponding to the given distance ΔX. In other words, the tool center point TCP2 may move a distance smaller than the distance corresponding to the given distance ΔX, while the virtual position P0 may move the distance corresponding to the given distance ΔX.

The functionality of the elements disclosed herein may be implemented using circuitry or processing circuitry that includes general purpose processors, special purpose processors, integrated circuits, application specific integrated circuits (ASICs), conventional circuitry and/or combinations thereof that are configured or programmed to perform the disclosed functionality. Processors are considered processing circuitry or circuitry as they include transistors and other circuitry therein. In the present disclosure, the circuitry, units, or means are hardware that carries out the recited functionality or hardware that is programmed to perform the recited functionality. The hardware may be hardware disclosed herein or other known hardware that is programmed or configured to carry out the recited functionality. When the hardware is a processor that may be considered a type of circuitry, the circuitry, means, or units are a combination of hardware and software, and the software is used to configure the hardware and/or processor.

### Aspects

It will be appreciated by those skilled in the art that the exemplary embodiments described above are specific examples of the following aspects.

### (Item 1)

A robotic surgical system comprising:
a surgical apparatus including a first robot arm to support an endoscope;
an operation apparatus including an operation unit to receive an operation for the endoscope; and
a controller configured or programmed to perform a process to move a control point set at a virtual position offset ahead of a distal end of the endoscope in an axial direction of the endoscope by a distance corresponding to a given distance when the operation unit receives an operation to move the endoscope by the given distance.

### (Item 2)

The robotic surgical system according to item 1, wherein the controller is configured or programmed to perform the process to move the control point set at the virtual position by the distance corresponding to the given distance with a pivot position, which serves as a fulcrum for movement of the endoscope, as a fulcrum when the operation unit receives the operation to move the endoscope by the given distance.

### (Item 3)

The robotic surgical system according to item 2, wherein
the first robot arm is configured to rotate the endoscope around a longitudinal axis of the endoscope; and
the control point set at the virtual position is set on the longitudinal axis.

### (Item 4)

The robotic surgical system according to item 2 or 3, wherein the controller is configured or programmed to perform the process to move the control point set at the virtual position by the distance corresponding to the given distance based on a relational expression ΔX1 = ΔX × L1/(L1 + L2), where ΔX represents the given distance, ΔX1 represents the distance corresponding to the given distance, L1 represents a distance from the pivot position to the distal end of the endoscope, and L2 represents a distance from the distal end of the endoscope to the virtual position, when the operation unit receives the operation to move the endoscope by the given distance.

### (Item 5)

The robotic surgical system according to any one of items 1 to 4, wherein
the surgical apparatus includes a second robot arm to support an instrument;
the operation unit is configured to receive an operation for the instrument; and
the controller is configured or programmed to perform a process to move a control point set at a distal end of the instrument by the distance corresponding to the given distance when the operation unit receives an operation to move the instrument by the given distance.

### (Item 6)

The robotic surgical system according to item 5, wherein the controller is configured or programmed to perform the process to move the control point by the distance corresponding to the given distance with a pivot position, which serves as a fulcrum for movement of the instrument, as a fulcrum when the operation unit receives the operation to move the instrument by the given distance.

### (Item 7)

The robotic surgical system according to item 5 or 6, wherein
the instrument includes an end effector, a first support member to support the end effector such that the end effector is rotatable around a first rotation axis, a second support member to support the first support member such that the first support member is rotatable around a second rotation axis, and a shaft to support the second support member; and
the control point is set at a distal end of the end effector, an intersection of a longitudinal axis of the shaft with the first rotation axis, or an intersection of the longitudinal axis of the shaft with the second rotation axis.

### (Item 8)

The robotic surgical system according to any one of items 5 to 7, wherein the controller is configured or programmed to:
perform a process to scale a received operation amount and actually move the instrument when the operation unit receives an operation to move the instrument; and
perform a process to scale the received operation amount by a same amount as when the instrument is moved and actually move the endoscope when the operation unit receives an operation to move the endoscope.

### (Item 9)

The robotic surgical system according to any one of items 5 to 8, wherein a distance of the virtual position from the distal end of the endoscope is set based on a distance between the control point of the instrument and the distal end of the endoscope.

### (Item 10)

The robotic surgical system according to any one of items 5 to 9, wherein
the operation unit includes:
   a right-handed operation unit to be operated by a right hand of an operator; and
   a left-handed operation unit to be operated by a left hand of the operator;
an operation to move the instrument is received by one of the right-handed operation unit and the left-handed operation unit; and
an operation to move the endoscope is received by both the right-handed operation unit and the left-handed operation unit.

### (Item 11)

The robotic surgical system according to any one of items 1 to 4, wherein the controller is configured or programmed to perform a process to scale a received operation amount and actually move the endoscope when the operation unit receives an operation to move the endoscope.

### (Item 12)

A control method for a robotic surgical system, the control method comprising:
receiving, by an operation unit configured to receive an operation for an endoscope supported by a robot arm, an operation to move the endoscope by a given distance; and
moving a control point set at a virtual position offset ahead of a distal end of the endoscope in an axial direction of the endoscope by a distance corresponding to the given distance.

### (Item 13)

A program for the control method for a robotic surgical system according to item 12.

### (Item 14)

A storage medium configured to store the program for the control method for a robotic surgical system according to item 13.

## Claims

1. A robotic surgical system (500) comprising:
a surgical apparatus (100) including a first robot arm (50c) to support an endoscope (3);
an operation apparatus (200) including an operation unit (110) to receive an operation for the endoscope; and
a controller (310) configured or programmed to perform a process to move a control point set at a virtual position (P0) offset ahead of a distal end of the endoscope in an axial direction of the endoscope by a distance corresponding to a given distance (ΔX) when the operation unit receives an operation to move the endoscope by the given distance.

2. The robotic surgical system according to claim 1, wherein the controller is configured or programmed to perform the process to move the control point set at the virtual position by the distance corresponding to the given distance with a pivot position (PP2), which serves as a fulcrum for movement of the endoscope, as a fulcrum when the operation unit receives the operation to move the endoscope by the given distance.

3. The robotic surgical system according to claim 2, wherein
the first robot arm is configured to rotate the endoscope around a longitudinal axis of the endoscope; and
the control point set at the virtual position is set on the longitudinal axis.

4. The robotic surgical system according to claim 2 or 3, wherein the controller is configured or programmed to perform the process to move the control point set at the virtual position by the distance corresponding to the given distance based on a relational expression ΔX1 = ΔX × L1/(L1 + L2), where ΔX represents the given distance, ΔX1 represents the distance corresponding to the given distance, L1 represents a distance from the pivot position to the distal end of the endoscope, and L2 represents a distance from the distal end of the endoscope to the virtual position, when the operation unit receives the operation to move the endoscope by the given distance.

5. The robotic surgical system according to any one of claims 1 to 4, wherein
the surgical apparatus includes a second robot arm (50a, 50b, 50d) to support an instrument (2);
the operation unit is configured to receive an operation for the instrument; and
the controller is configured or programmed to perform a process to move a control point set at a distal end of the instrument by the distance corresponding to the given distance when the operation unit receives an operation to move the instrument by the given distance.

6. The robotic surgical system according to claim 5, wherein the controller is configured or programmed to perform the process to move the control point by the distance corresponding to the given distance with a pivot position (PP1), which serves as a fulcrum for movement of the instrument, as a fulcrum when the operation unit receives the operation to move the instrument by the given distance.

7. The robotic surgical system according to claim 5 or 6, wherein
the instrument includes an end effector (2b), a first support member (2f) to support the end effector such that the end effector is rotatable around a first rotation axis (A11), a second support member (2e) to support the first support member such that the first support member is rotatable around a second rotation axis (A10), and a shaft to support the second support member; and
the control point is set at a distal end of the end effector, an intersection of a longitudinal axis of the shaft with the first rotation axis, or an intersection of the longitudinal axis of the shaft with the second rotation axis.

8. The robotic surgical system according to any one of claims 5 to 7, wherein the controller is configured or programmed to:
perform a process to scale a received operation amount and actually move the instrument when the operation unit receives an operation to move the instrument; and
perform a process to scale the received operation amount by a same amount as when the instrument is moved and actually move the endoscope when the operation unit receives an operation to move the endoscope.

9. The robotic surgical system according to any one of claims 5 to 8, wherein a distance of the virtual position from the distal end of the endoscope is set based on a distance between the control point of the instrument and the distal end of the endoscope.

10. The robotic surgical system according to any one of claims 5 to 9, wherein
the operation unit includes:
a right-handed operation unit (110R) to be operated by a right hand of an operator; and
a left-handed operation unit (110L) to be operated by a left hand of the operator;
an operation to move the instrument is received by one of the right-handed operation unit and the left-handed operation unit; and
an operation to move the endoscope is received by both the right-handed operation unit and the left-handed operation unit.

11. The robotic surgical system according to any one of claims 1 to 4, wherein the controller is configured or programmed to perform a process to scale a received operation amount and actually move the endoscope when the operation unit receives an operation to move the endoscope.

12. A control method for a robotic surgical system (500), the control method comprising:
receiving, by an operation unit (110) configured to receive an operation for an endoscope (3) supported by a robot arm (50c), an operation to move the endoscope by a given distance; and
moving a control point set at a virtual position (P0) offset ahead of a distal end of the endoscope in an axial direction of the endoscope by a distance corresponding to the given distance.

13. A program for the control method for a robotic surgical system (500) according to claim 12.

14. A storage medium configured to store the program for the control method for a robotic surgical system (500) according to claim 13.
